# EUROPEAN PATENT APPLICATION

(11) **EP 4 763 238 A1**
(43) Date of publication of application: **24.06.2026**
(21) Application number: 25814130.8
(22) Date of filing: 20.01.2025
(51) Int. Cl.: A61L 27/12, A61L 27/02, A61L 27/50

(54) **BIOMIMETIC BONE WITH CONTROLLABLE SOLIDIFICATION TIME, PREPARATION METHOD THEREFOR, AND USE THEREOF**

(30) Priority: 30.05.2024 CN 202410688887
(71) Applicant: Hangzhou Origo Biotechnology Co. Ltd., Hangzhou, Zhejiang 310000 (CN)
(72) Inventor: LIN, Xianfeng, Hangzhou, Zhejiang 310000 (CN); GU, Chenhui, Hangzhou, Zhejiang 310000 (CN); FAN, Shunwu, Hangzhou, Zhejiang 310000 (CN); TIAN, Hongsen, Hangzhou, Zhejiang 310000 (CN); CHEN, Pengfei, Hangzhou, Zhejiang 310000 (CN)
(74) Representative: Finetti, Claudia
(86) International application number: PCT/CN2025/073276
(87) International publication number: WO 2025/246405

(57) **Abstract**

The present invention belongs to the field of bone repair biomaterials. Disclosed in the present invention is a biomimetic bone with a controllable solidification time, including a calcium phosphate salt, a phosphoric acid-based organic matter, and a biomimetic cancellization reaction agent. The biomimetic cancellization reaction agent includes a cancellous reaction liquid phase and a cancellization reaction solid phase. The cancellization reaction liquid phase includes water, sodium citrate, and citric acid. The cancellization reaction solid phase is sodium bicarbonate. The biomimetic bone of the present invention can effectively control the solidification time and the degree of cancellization. Different ratios can be selected according to the requirements of clinical operation time and the cancellization condition of the clinical filling site, making the biomimetic bone easy for clinical use.

## Description

### Cross-Reference to Related Application

This application claims the benefit of Chinese Patent Application No. 202410688887.1, filed on May 30, 2024, the disclosure of which is hereby incorporated by reference in its entirety.

### Technical Field

The present invention belongs to the field of bone repair biomaterials, and specifically relates to a biomimetic bone with a controllable solidification time, a preparation method therefor, and use thereof.

### Background

The primary treatment strategies for bone fractures and bone defects are reduction, fixation, and functional exercise. At present, clinical fixation and filling of bone fractures and bone defects mainly rely on internal fixation and bone graft materials, including autologous bone, allogeneic bone, and xenogeneic decellularized bone, followed by osseointegration and repair under the self-regenerative capability of the body. However, this treatment approach has two subsequent drawbacks: firstly, a secondary surgical procedure is often required to remove the internal fixation; and secondly, the integration of the bone graft material with the host bone requires a considerable amount of time. Therefore, there is an urgent need to develop a novel biomedical material for immediate repair of bone tissue structures for clinical treatment of bone fractures and bone defects.

For immediate bone tissue repair, some solutions have been proposed. Firstly, bone adhesives are considered an effective solution for repairing cortical bone, as they form strong adhesion at fracture ends, restore the physiological position of the bone, and achieve skeletal support and stress strength. Patent Application No. CN202310767115.2 discloses an injectable adhesive for promoting fracture healing. The adhesive mimics a marine barnacle-derived biomaterial, where carboxyl groups on side chains thereof can absorb interfacial water to disrupt the binding between the interface and the water layer, thereby enhancing the binding strength between the dynamic network and the interface and thus improving the adhesion performance of the adhesive. Although the adhesive has good adhesion performance in solution, its composition differs significantly from that of bone, and issues remain with respect to mechanical strength and biosafety. Patent Application No. CN202211370224.2 discloses a photocurable bone adhesive, in which a photoinitiator is added to the bone adhesive to reduce the solidification time of the bone adhesive. However, the solidification process of the adhesive requires light irradiation for initiation, thereby reducing the convenience of use of the adhesive. Secondly, artificial bones are another solution, as they may mimic natural bone tissue structures, thereby promoting bone ingrowth and integration. Patent Application No. CN200910043405.2 discloses a method for manufacturing a biomimetic bone material with non-uniform pore distribution. Using HA/Ti or HA/316L composite powder as a raw material, the raw material powder is separated in a mold by molybdenum sheets to form a dense layer/transition layer/porous layer, followed by conventional compaction to obtain a green body, which is then subjected to vacuum sintering to obtain the biomimetic bone. However, the composition and physical properties of the biomimetic bone differ significantly from those of bone tissue, consequently failing to achieve immediate integration with bone tissue. Patent Application No. CN202210656568.3 discloses a biomimetic bone material based on icariin-functionalized polylactic acid and a preparation method therefor, which similarly has significant compositional differences from bone tissue.

Therefore, there is an urgent need to develop a biomimetic bone with a composition close to that of bone and a controllable solidification time, which may achieve immediate bone repair.

### Summary

In order to overcome the defects and deficiencies existing in the related art, the present invention provides a biomimetic bone with a controllable solidification time, a preparation method therefor, and use thereof.

Specifically, the present invention is achieved through the technical solutions in the following aspects.

In a first aspect, the present invention provides a biomimetic bone with a controllable solidification time. The biomimetic bone with the controllable solidification time is a biomimetic bone obtained by mixing and reacting a calcium phosphate salt, a phosphoric acid-based organic matter, and a biomimetic cancellization reaction agent.

In some embodiments, a ratio of the calcium phosphate salt, the phosphoric acid-based organic matter, and the biomimetic cancellization reaction agent is 0.1 g-5 g: 0.05-3 g: 0.1-1 mL.

In some embodiments, the ratio of the calcium phosphate salt, the phosphoric acid-based organic matter, and the biomimetic cancellization reaction agent is 0.5 g-2 g:0.2-1 g:0.2-0.5 mL.

Preferably, the ratio of the calcium phosphate salt, the phosphoric acid-based organic matter, and the biomimetic cancellization reaction agent is 1 g:0.5 g:0.35 mL.

In some embodiments, the calcium phosphate salt is one or a combination of tetracalcium phosphate, calcium phosphate, and hydroxyapatite.

Preferably, the calcium phosphate salt is tetracalcium phosphate.

In some embodiments, the phosphoric acid-based organic matter is configured to integrate the calcium phosphate salt, and includes one or a combination of phosphoamino acid, phosphonocarboxylic acid, and bisphosphonate.

Preferably, the phosphoric acid-based organic matter is phosphoamino acid.

More preferably, the phosphoric acid-based organic matter is phosphoserine.

In some embodiments, the biomimetic cancellization reaction agent is formed by mixing a biomimetic cancellization liquid phase reaction agent and a biomimetic cancellization solid phase reaction agent.

The liquid phase reaction agent includes sodium citrate and/or citric acid, the liquid phase reaction agent further includes water, and the solid phase reaction agent is sodium bicarbonate.

Alternatively, optionally, the biomimetic cancellization reaction agent is sodium citrate.

Preferably, a molar ratio of sodium bicarbonate to citric acid is 3:1, and reaction products thereof are sodium citrate and carbon dioxide.

Preferably, a ratio of water, sodium citrate, and citric acid is 0.2-0.5 mL:0-500 mg:0-400 mg.

More preferably, the ratio of water, sodium citrate, and citric acid is 0.3-0.4 mL:0-155 mg:0-115 mg.

In a second aspect, the present invention provides a preparation method for the biomimetic bone with the controllable solidification time in the above first aspect, including the following steps.
(1) A calcium phosphate salt, a phosphoric acid-based organic matter, and sodium bicarbonate are separately weighed and uniformly mixed to prepare a solid phase component, and the solid phase component is loaded into a threaded disposable syringe for later use.
(2) Solid citric acid and solid sodium citrate are separately weighed and added to water to prepare a liquid phase component, and the liquid phase component is loaded into another threaded disposable syringe for later use.
(3) The two threaded disposable syringes prepared in S(1) and S(2), which include the solid phase component and the liquid phase component, respectively, are connected using a disposable connector, and then the solid phase component and the liquid phase component are rapidly and uniformly mixed to prepare a paste-like biomimetic bone liquid.
(4) The paste-like biomimetic bone liquid prepared in S(3) is injected into a target site for solidification.

In some embodiments, the threaded disposable syringe has a volume of 1-10 mL.

Preferably, a 5 mL threaded disposable syringe is used.

In some embodiments, the solidification time is 0-15 min. The solidification time may be adjusted according to the total content of sodium citrate.

As an optional implementation, in S(4), the contents of the two syringes may be uniformly mixed and then pushed into one of the syringes for injection.

In a third aspect, the present invention provides use of the biomimetic bone with the controllable solidification time in the above first aspect, or the biomimetic bone with the controllable solidification time prepared by the preparation method in the above second aspect.

Preferably, the present invention provides use of the biomimetic bone with the controllable solidification time in the above first aspect, or the biomimetic bone with the controllable solidification time prepared by the preparation method in the above second aspect, in the preparation of a bone repair biomaterial.

In some embodiments, a paste-like biomimetic bone fluid is injected into a target site using a disposable syringe for solidification.

In some embodiments, the biomimetic bone is applied for the adhesive filling of bone fractures and bone defects. The biomimetic bone of the present invention has certain compressive strength, tensile strength, and adhesion performance, and can effectively control the solidification time and the degree of cancellization. Different ratios can be selected according to the requirements of clinical operation time and the cancellization condition of the clinical filling site, making the biomimetic bone easy for clinical use.

In some embodiments, the biomimetic bone has compressive strength, with an index range of 1.0-10.0 MPa.

In some embodiments, the biomimetic bone has tensile strength, with an index range of 1.0-2.5 MPa.

In some embodiments, the biomimetic bone has adhesion strength, with an index range of 0.2-1.0 MPa.

In some embodiments, the biomimetic bone has an adjustable solidification time, with a time range of 10 s-900 s.

In some embodiments, the biomimetic bone has an adjustable solidification time, with a trabecular spacing of 45 µm-1000 µm, a bone area range of 53%-95%, and a bone volume range of 38%-96%.

The present invention has the following beneficial effects.
(1) According to the biomimetic bone of the present invention, the biomimetic bone is formed immediately through the reaction, which facilitates immediate integration of biomimetic bone and autologous bone during surgery.
(2) The biomimetic bone of the present invention may effectively control the solidification time by adjusting the ratio of sodium citrate, allowing selection according to the implantation time requirements of different surgical sites during surgery, thereby preventing premature or delayed solidification.
(3) The biomimetic bone of the present invention may effectively control the degree of cancellization by adjusting the ratio of the biomimetic cancellization reaction agent, so as to adapt to the mechanical strength and trabecular density of the surgical implantation site.

### Brief Description of the Drawings

In order to make the objectives, technical solutions, and beneficial effects of the present invention clearer, the present invention is illustrated by the following drawings.
Fig. 1 is a diagram of a bone surface of a biomimetic bone observed under a scanning electron microscope.
Fig. 2 is an image showing trabecular density of a biomimetic bone reconstructed by Micro-CT.
Fig. 3 is an image of MC3T3 cells cultured with an extract of a biomimetic bone, osteogenically induced using an osteogenic induction medium, and subsequently stained with an alkaline phosphatase staining kit.
Fig. 4 shows tube formation promoted by Human Umbilical Vein Endothelial Cells (HUVEC) cultured with an extract of a biomimetic bone.
Fig. 5 shows maintenance of bone continuity and osseointegration, reconstructed by Micro-CT, after use of a biomimetic bone in a rabbit comminuted fracture model.
Fig. 6 shows osseointegration, reconstructed by Micro-CT, after use of a biomimetic bone in a rabbit defect model.

### Detailed Description of the Embodiments

A biomimetic bone with a controllable solidification time, a preparation method therefor, and use thereof provided by the present invention will be described below in detail in conjunction with the embodiments. However, these embodiments should not be construed as limiting the scope of protection of the present invention.

### Embodiment 1

1.0 g of tetracalcium phosphate and 0.5 g of phosphoserine were weighed and uniformly mixed to prepare a solid phase component, and the solid phase component was loaded into a 5 mL threaded disposable syringe for later use. 0.35 mL of water was used as a liquid phase component and loaded into another 5 mL threaded disposable syringe for later use. The above two threaded disposable syringes, which included the solid phase component and the liquid phase component, respectively, were connected using a disposable connector and rapidly and uniformly mixed to prepare a paste-like biomimetic bone liquid.

### Embodiment 2

1.0 g of tetracalcium phosphate, 0.5 g of phosphoserine, and 76 mg of sodium bicarbonate were weighed and uniformly mixed to prepare a solid phase component, and the solid phase component was loaded into a 5 mL threaded disposable syringe for later use. 58 mg of citric acid was added to 0.35 mL of water to prepare a liquid phase component, and the liquid phase component was loaded into another 5 mL threaded disposable syringe for later use. The above two threaded disposable syringes, which included the solid phase component and the liquid phase component, respectively, were connected using a disposable connector and rapidly and uniformly mixed to prepare a paste-like biomimetic bone liquid.

### Embodiment 3

1.0 g of tetracalcium phosphate, 0.5 g of phosphoserine, and 38 mg of sodium bicarbonate were weighed and uniformly mixed to prepare a solid phase component, and the solid phase component was loaded into a 5 mL threaded disposable syringe for later use. 39 mg of sodium citrate and 29 mg of citric acid were added to 0.35 mL of water to prepare a liquid phase component, and the liquid phase component was loaded into another 5 mL threaded disposable syringe for later use. The above two threaded disposable syringes, which included the solid phase component and the liquid phase component, respectively, were connected using a disposable connector and rapidly and uniformly mixed to prepare a paste-like biomimetic bone liquid.

### Embodiment 4

1.0 g of tetracalcium phosphate and 0.5 g of phosphoserine were weighed and uniformly mixed to prepare a solid phase component, and the solid phase component was loaded into a 5 mL threaded disposable syringe for later use. 78 mg of sodium citrate was added to 0.35 mL of water to prepare a liquid phase component, and the liquid phase component was loaded into another 5 mL threaded disposable syringe for later use. The above two threaded disposable syringes, which included the solid phase component and the liquid phase component, respectively, were connected using a disposable connector and rapidly and uniformly mixed to prepare a paste-like biomimetic bone liquid.

### Embodiment 5

1.0 g of tetracalcium phosphate, 0.5 g of phosphoserine, and 151 mg of sodium bicarbonate were weighed and uniformly mixed to prepare a solid phase component, and the solid phase component was loaded into a 5 mL threaded disposable syringe for later use. 115 mg of sodium citrate was added to 0.35 mL of water to prepare a liquid phase component, and the liquid phase component was loaded into another 5 mL threaded disposable syringe for later use. The above two threaded disposable syringes, which included the solid phase component and the liquid phase component, respectively, were connected using a disposable connector and rapidly and uniformly mixed to prepare a paste-like biomimetic bone liquid.

### Embodiment 6

1.0 g of tetracalcium phosphate, 0.5 g of phosphoserine, and 76 mg of sodium bicarbonate were weighed and uniformly mixed to prepare a solid phase component, and the solid phase component was loaded into a 5 mL threaded disposable syringe for later use. 78 mg of sodium citrate and 58 mg of citric acid were added to 0.35 mL of water to prepare a liquid phase component, and the liquid phase component was loaded into another 5 mL threaded disposable syringe for later use. The above two threaded disposable syringes, which included the solid phase component and the liquid phase component, respectively, were connected using a disposable connector and rapidly and uniformly mixed to prepare a paste-like biomimetic bone liquid.

### Embodiment 7

1.0 g of tetracalcium phosphate and 0.5 g of phosphoserine were weighed and uniformly mixed to prepare a solid phase component, and the solid phase component was loaded into a 5 mL threaded disposable syringe for later use. 155 mg of sodium citrate was added to 0.35 mL of water to prepare a liquid phase component, and the liquid phase component was loaded into another 5 mL threaded disposable syringe for later use. The above two threaded disposable syringes, which included the solid phase component and the liquid phase component, respectively, were connected using a disposable connector and rapidly and uniformly mixed to prepare a paste-like biomimetic bone liquid.

### Embodiment 8

1.0 g of hydroxyapatite, 0.5 g of phosphoserine, and 38 mg of sodium bicarbonate were weighed and uniformly mixed to prepare a solid phase component, and the solid phase component was loaded into a 5 mL threaded disposable syringe for later use. 39 mg of sodium citrate and 29 mg of citric acid were added to 0.35 mL of water to prepare a liquid phase component, and the liquid phase component was loaded into another 5 mL threaded disposable syringe for later use. The above two threaded disposable syringes, which included the solid phase component and the liquid phase component, respectively, were connected using a disposable connector and rapidly and uniformly mixed to prepare a paste-like biomimetic bone liquid.

### Embodiment 9

1.0 g of tetracalcium phosphate, 0.5 g of phosphotyrosine, and 38 mg of sodium bicarbonate were weighed and uniformly mixed to prepare a solid phase component, and the solid phase component was loaded into a 5 mL threaded disposable syringe for later use. 39 mg of sodium citrate and 29 mg of citric acid were added to 0.35 mL of water to prepare a liquid phase component, and the liquid phase component was loaded into another 5 mL threaded disposable syringe for later use. The above two threaded disposable syringes, which included the solid phase component and the liquid phase component, respectively, were connected using a disposable connector and rapidly and uniformly mixed to prepare a paste-like biomimetic bone liquid.

### Embodiment 10

1.0 g of tetracalcium phosphate, 0.5 g of 3-phosphonopropionic acid, and 38 mg of sodium bicarbonate were weighed and uniformly mixed to prepare a solid phase component, and the solid phase component was loaded into a 5 mL threaded disposable syringe for later use. 39 mg of sodium citrate and 29 mg of citric acid were added to 0.35 mL of water to prepare a liquid phase component, and the liquid phase component was loaded into another 5 mL threaded disposable syringe for later use. The above two threaded disposable syringes, which included the solid phase component and the liquid phase component, respectively, were connected using a disposable connector and rapidly and uniformly mixed to prepare a paste-like biomimetic bone liquid.

The biomimetic bone obtained from Embodiments 1-10 was subjected to solidification time, compressive strength, tensile strength, and adhesion strength testing, respectively.

Effect Embodiment 1: Solidification Time, Compressive Strength, Tensile Strength, and Adhesion Strength Testing
1. The paste-like biomimetic bone liquid obtained from Embodiments 1-10 was extruded into a mold with a diameter of 6 mm for solidification. The solidification time was recorded. After solidification, the obtained biomimetic bone was removed and subjected to compressive strength testing.
2. The paste-like biomimetic bone liquid obtained from Embodiments 1-10 was uniformly applied to two fractured ends of porcine femurs. Starting from the adhesion, a 10 N force was applied on top of a metal post. After solidification, a test specimen was placed in a fixture, and the tensile strength was tested. The applied force was perpendicular to the surface of the adhered porcine bone at a 90°. A universal joint or metal wire was used to connect a testing machine and the adhered porcine bone. The tensile strength of the adhered porcine femur was tested at a speed of 20 mm/min.
3. The paste-like biomimetic bone liquid obtained from Embodiments 1-10 was applied to a smooth porcine bone slice. A 10 N force was applied to press the slice for solidification. After solidification, the test specimen was incubated at 37 °C for 30 minutes, then placed in a fixture, and the adhesion strength was tested. The applied force was parallel to the adhered porcine bone slice. The adhesion strength of the adhered porcine bone slice was tested at a speed of 20 mm/min.

The test results are shown in Table 1.

**Table 1**

| Sequence number | Calcium phosphate salt | Phosphoric acid-based organic matter | Ratio of pore-forming reaction agent | Total sodium citrate | Solidification time (s) | Compressive strength (MPa) | Tensile strength (MPa) | Adhesion strength (MPa) |
|---|---|---|---|---|---|---|---|---|
| Embodiment 1 | Tetracalcium phosphate | Phospho serine | Sodium citrate: 0 mg, citric acid 0 mg | 0 mg | 25±6 | 8±2 | 1.21±0.62 | 0.48±0.12 |
| | | | Sodium bicarbonate: 0 mg | | | | | |
| Embodiment 2 | Tetracalcium phosphate | Phospho serine | Sodium citrate: 0 mg, citric acid 58 mg | 78 mg | 160± 36 | 1±1 | 1.32±0.75 | 0.51±0.22 |
| | | | Sodium bicarbonate: 76 mg | | | | | |
| Embodiment 3 | Tetracalcium phosphate | Phospho serine | Sodium citrate: 39 mg, citric acid 29 mg | 78 mg | 200± 44 | 2±1 | 1.45±0.87 | 0.67±0.17 |
| | | | Sodium bicarbonate: 38 mg | | | | | |
| Embodiment 4 | Tetracalcium phosphate | Phospho serine | Sodium citrate: 78 mg, citric acid 0 mg | 78 mg | 240± 57 | 8±2 | 1.95±0. 33 | 0.63±0. 24 |
| | | | Sodium bicarbonate: 0 mg | | | | | |
| Embodiment 5 | Tetracalcium phosphate | Phospho serine | Sodium citrate: 0 mg, citric acid 115 mg | 155 mg | 565± 97 | 1±1 | 1.14±0. 56 | 0.23±0. 14 |
| | | | Sodium bicarbonate: 151 mg | | | | | |
| Embodiment 6 | Tetracalcium phosphate | Phospho serine | Sodium citrate: 78 mg, citric acid 58 mg | 155 mg | 630± 106 | 4±3 | 1.26±0. 39 | 0.56±0. 16 |
| | | | Sodium bicarbonate: 76 mg | | | | | |
| Embodiment 7 | Tetracalcium phosphate | Phospho serine | Sodium citrate: 155 mg, citric acid 0 mg | 155 mg | 710±119 | 4±3 | 1.02±0.83 | 0.37±0.23 |
| | | | Sodium bicarbonate: 0 mg | | | | | |
| Embodiment 8 | Hydroxyapatite | Phospho serine | Sodium citrate: 39 mg, citric acid 29 mg | 78 mg | 195± 51 | 2±1 | 1.34±0. 72 | 0.62±0. 21 |
| | | | Sodium bicarbonate: 38 mg | | | | | |
| Embodiment 9 | Tetracalcium phosphate | Phosphot yrosine | Sodium citrate: 39 mg, citric acid 29 mg | 78 mg | 310±45 | 2±1 | 1.24±0.65 | 0.54±0.31 |
| | | | Sodium bicarbonate: 38 mg | | | | | |
| Embodi ment 10 | Tetracalcium phosphate | 3-phosph onopropionic acid | Sodium citrate: 39 mg, citric acid 29 mg | 78 mg | 420±63 | 2±1 | 1.07±0.72 | 0.51±0.27 |
| | | | Sodium bicarbonate: 38 mg | | | | | |

It was observed from the table that, as the total content of sodium citrate increased, the solidification time correspondingly increased. Meanwhile, as the ratio of citric acid increased, the compressive strength and tensile strength of the biomimetic bone decreased, while the adhesion strength showed no significant trend.

Effect Embodiment 2: Bone Parameter Testing
1. The biomimetic bone obtained from Embodiments 1-7 was examined by scanning electron microscopy to observe the size and number of pores, and the trabecular spacing and bone area percentage were counted.
2. The biomimetic bone obtained from Embodiments 1-7 was scanned using micro-CT, followed by 3D reconstruction, and the bone volume percentage was counted.

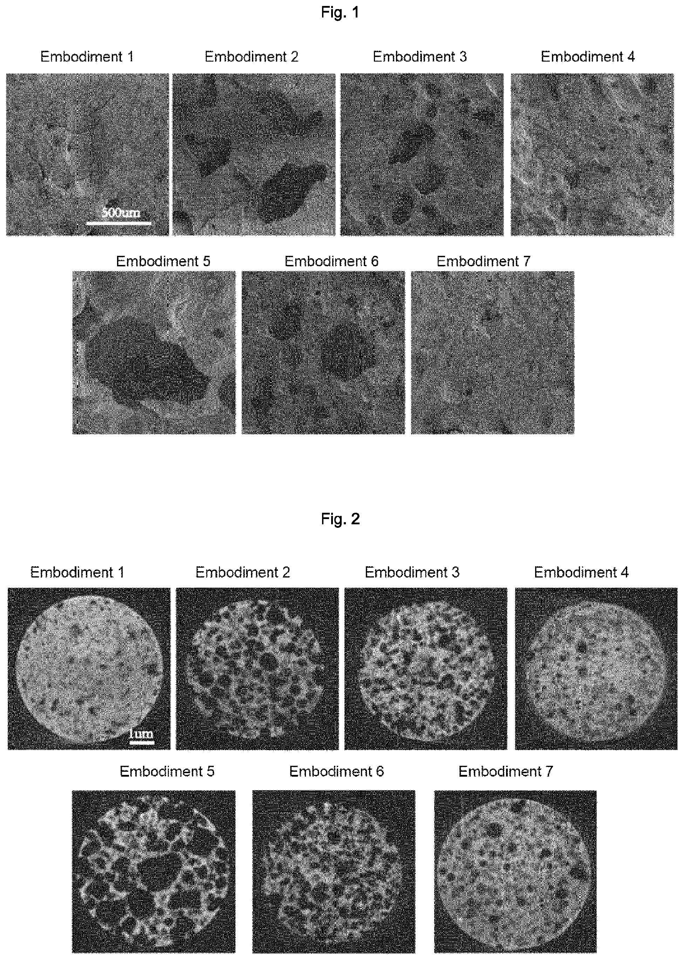

The test results are shown in Fig. 1, Fig. 2, and Table 2.

**Table 2**

| Sequence number | Ratio of pore-forming reaction agent | Total sodium citrate | Trabecular spacing size (µm) | Bone area (%) | Bone volume (%) |
|---|---|---|---|---|---|
| Embodiment 1 | Sodium citrate: 0 mg, citric acid 0 mg | 0 mg | 86.7±32.5 | 91.9±2.3 | 95.9±1.0 |
| | Sodium bicarbonate: 0 mg | | | | |
| Embodiment 2 | Sodium citrate: 0 mg, citric acid 58 mg | 78 mg | 497.6±102.2 | 78.6±8.6 | 62.5±9.7 |
| | Sodium bicarbonate: 76 mg | | | | |
| Embodiment 3 | Sodium citrate: 39 mg, citric acid 29 mg | 78 mg | 314.7±98.3 | 82.6±6.7 | 68.2±6.9 |
| | Sodium bicarbonate: 38 mg | | | | |
| Embodiment 4 | Sodium citrate: 78 mg, citric acid 0 mg | 78 mg | 67.4±43.1 | 93.6±2.4 | 94.4±1.2 |
| | Sodium bicarbonate: 0 | | | | |
| | mg | | | | |
| Embodiment 5 | Sodium citrate: 0 mg, citric acid 115 mg | 155 mg | 987.6±684.6 | 53.3±9.2 | 38.9±9.0 |
| | Sodium bicarbonate: 151 mg | | | | |
| Embodiment 6 | Sodium citrate: 78 mg, citric acid 58 mg | 155 mg | 443.1±148.3 | 80.6±10.3 | 60.9±8.5 |
| | Sodium bicarbonate: 76 mg | | | | |
| Embodiment 7 | Sodium citrate: 155 mg, citric acid 0 mg | 155 mg | 47.2±33.7 | 94.7±4.4 | 91.8±1.5 |
| | Sodium bicarbonate: 0 mg | | | | |

It was observed from the table that, as the ratio of citric acid increased, the trabecular spacing correspondingly increased, while the bone area percentage and bone volume percentage decreased, indicating that the degree of cancellization of the biomimetic bone was controlled by adjusting the ratio of citric acid.

Effect Embodiment 3: Osteogenesis Testing, Tube Formation Testing, and Osseointegration Testing

### 3.1 Osteogenesis Testing

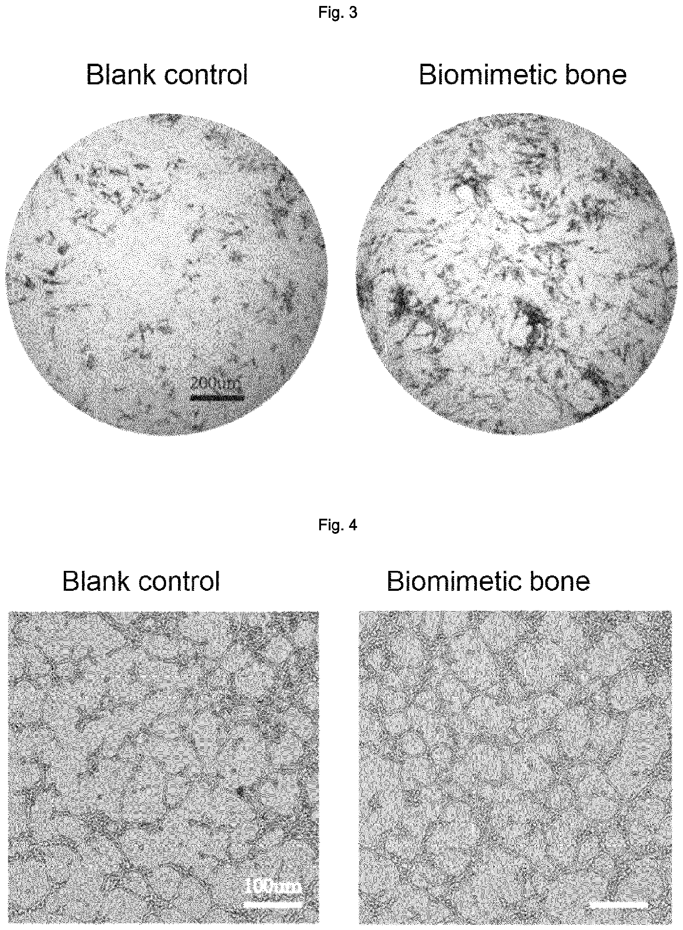

An extract of the biomimetic bone was used for an osteogenic differentiation experiment. The specific procedure was as follows: MC3T3 cells (from American Type Culture Collection (ATCC)) were seeded into a 96-well plate at a density of 5,000 cells per well. The extract obtained from Embodiment 4 (test group) or regular α-MEM medium (control group) was added to different wells. Meanwhile, an equal volume of osteogenic induction medium (10 mmol/L β-glycerophosphate, 0.05 mmol/L vitamin C, and 100 mmol/L dexamethasone) was added to each well. The cells were cultured for 7 days, with the culture medium being replaced every 2 days. After 7 days, the cells were fixed with 4% paraformaldehyde for 10 minutes, and Alkaline Phosphatase (ALP) staining was performed using an ALP staining kit. The effect on the induction of osteogenic cell differentiation was determined according to the ALP staining area. The results of osteogenic differentiation are shown in Fig. 3. As shown in the figure, the biomimetic bone extract group exhibited more pronounced ALP staining compared with the blank control group, indicating effective promotion of osteogenesis.

### 3.2 Tube Formation Testing

50 µL of Matrigel was pre-coated onto the bottom of the 96-well plate at 4 °C, minimizing bubble formation during the process, and left overnight at 4 °C to allow uniform coating. The overnight 96-well plate was placed in a 37 °C incubator to allow the Matrigel to solidify. HUVEC cells (from ATCC) were trypsinized and resuspended in either regular Dulbecco's Modified Eagle Medium (DMEM) (control group) or the extract (test group) from Embodiment 4. The resuspended cells were seeded onto the Matrigel-coated 96-well plate at a density of 30,000 cells per well. After 6 hours of culture, tube formation of the cells was observed under a microscope. The results of tube formation testing are shown in Fig. 4. As shown in the figure, the biomimetic bone extract group exhibited more pronounced tube formation compared with the blank control group, indicating effective promotion of tube formation.

### 3.3 Cortical Osseointegration Testing

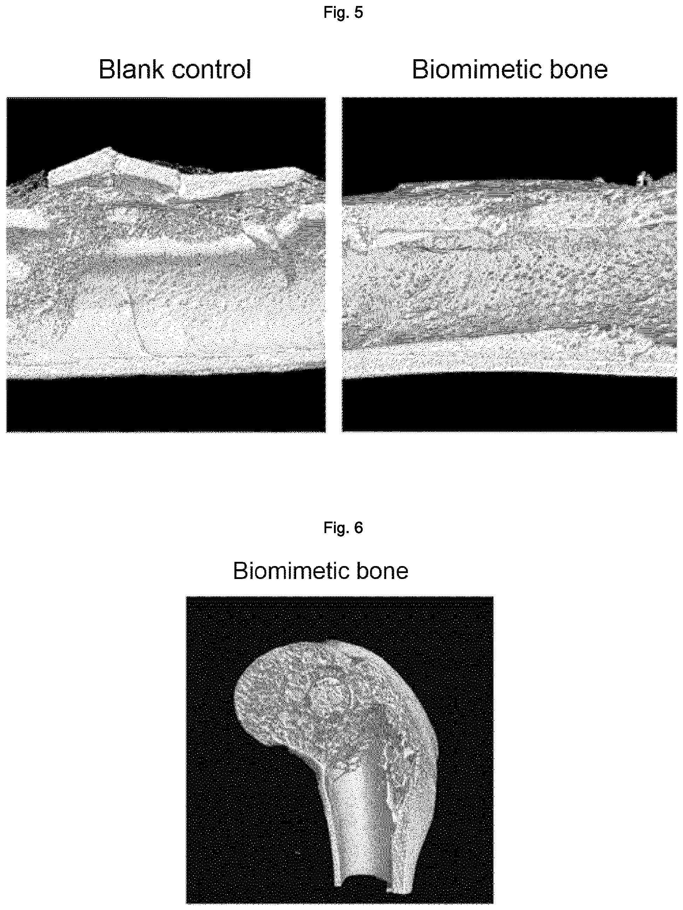

A comminuted fracture model was created in a rabbit femur. After anesthetizing the rabbit, the skin on the leg was shaved, disinfected, and incised. The tissues were dissected layer by layer to expose the femur. A rectangular bone defect model was created using a wire saw, and the bone fragment was crushed into four pieces. During surgery, the cortical bone fragments in the test group were adhered using the biomimetic bone from Embodiment 4, while no adhesion was applied in the control group. 4 weeks later, Micro-CT scanning and bone reconstruction were performed to observe whether the biomimetic bone was effectively adhered to and integrated with the cortical bone tissue. Micro-CT reconstruction results are shown in Fig. 5. As shown in the figure, in the biomimetic bone group compared with the control group, the cortical bone of the femur was effectively adhered and integrated, with continuous cortical bone, and the biomimetic bone was well integrated with the cortical bone, indicating excellent cortical osseointegration.

### 3.4 Cancellous Osseointegration Testing

A circular defect model was created in the femoral condyle of the rabbit. After anesthetizing the rabbit, the skin on the leg was shaved, disinfected, and incised. The tissues were dissected layer by layer to expose the femoral condyle. A circular bone defect was created using a punch. The biomimetic bone from Embodiment 4 was implanted into the defect site. 4 weeks later, Micro-CT scanning and bone reconstruction were performed to observe whether the biomimetic bone was integrated with the bone tissue. Micro-CT reconstruction results are shown in Fig. 6. As shown in the figure, the biomimetic bone was completely integrated with the cancellous bone at the defect site in the femoral condyle, with no visible interface, indicating excellent cancellous osseointegration.

The above are preferred implementations of the present invention, and it is to be noted that, although the present invention has been described in detail through the above preferred embodiments, those skilled in the art should understand that various modifications and refinements may still be made without departing from the principle of the present invention, and these improvements and refinements should also be considered within the scope of protection of the present invention, without departing from the scope defined by the claims of the present invention.

## Claims

1. A biomimetic bone with a controllable solidification time, wherein the biomimetic bone with the controllable solidification time is a biomimetic bone obtained by mixing and reacting a calcium phosphate salt, a phosphoric acid-based organic matter, and a biomimetic cancellization reaction agent.

2. The biomimetic bone according to claim 1, wherein a ratio of the calcium phosphate salt, the phosphoric acid-based organic matter, and the biomimetic cancellization reaction agent is 0.1 g-5 g:0.05-3 g:0.1-1 mL.

3. The biomimetic bone according to claim 1, wherein the calcium phosphate salt is one or a combination of tetracalcium phosphate, calcium phosphate, and hydroxyapatite, and the phosphoric acid-based organic matter is configured to integrate the calcium phosphate salt, and comprises one or a combination of phosphoamino acid, phosphonocarboxylic acid, and bisphosphonate.

4. The biomimetic bone according to claim 1, wherein the biomimetic cancellization reaction agent is formed by mixing a biomimetic cancellization liquid phase reaction agent and a biomimetic cancellization solid phase reaction agent.

5. The biomimetic bone according to claim 4, wherein the liquid phase reaction agent comprises sodium citrate and/or citric acid, the liquid phase reaction agent further comprises water, and the solid phase reaction agent is sodium bicarbonate, or the biomimetic cancellization reaction agent is sodium citrate.

6. The biomimetic bone according to claim 5, wherein a molar ratio of sodium bicarbonate to citric acid is 3:1, and reaction products thereof are sodium citrate and carbon dioxide;
preferably, a ratio of water, sodium citrate, and citric acid is 0.2-0.5 mL:0-500 mg:0-400 mg.

7. A preparation method for the biomimetic bone with the controllable solidification time according to any one of claims 1 to 6, comprising the following steps:
(1) separately weighing a calcium phosphate salt, a phosphoric acid-based organic matter, and sodium bicarbonate, uniformly mixing them to prepare a solid phase component, and loading the solid phase component into a threaded disposable syringe for later use;
(2) separately weighing solid citric acid and solid sodium citrate, adding them to water to prepare a liquid phase component, and loading the liquid phase component into another threaded disposable syringe for later use;
(3) connecting the two threaded disposable syringes prepared in S(1) and S(2), which comprise the solid phase component and the liquid phase component, respectively, using a disposable connector, and then rapidly and uniformly mixing the solid phase component and the liquid phase component to prepare a paste-like biomimetic bone liquid; and
(4) injecting the paste-like biomimetic bone liquid prepared in S(3) into a target site for solidification.

8. Use of the biomimetic bone with the controllable solidification time according to any one of claims 1 to 6, or the biomimetic bone with the controllable solidification time prepared by the preparation method according to claim 7, in the preparation of a bone repair biomaterial.

9. The use according to claim 8, wherein the biomimetic bone is applied for the adhesive filling of bone fractures and bone defects.

10. The use according to claim 8, wherein the biomimetic bone has compressive strength, with an index range of 1.0-10.0 MPa; the biomimetic bone has tensile strength, with an index range of 1.0-2.5 MPa; the biomimetic bone has adhesion performance, with an index range of 0.2-1.0 MPa; and the biomimetic bone has an adjustable solidification time, with a time range of 10 s-900 s.
